# EUROPEAN PATENT SPECIFICATION

(11) **EP 4 525 761 B1**
(45) Date of publication and mention of the grant of the patent: **04.06.2025**
(21) Application number: 24710515.8
(22) Date of filing: 04.03.2024
(51) Int. Cl.: A61B 18/14

(54) **KNIFE DESIGNS FOR END EFFECTORS USED IN SURGICAL TOOLS**
MESSER FÜR ENDEFFEKTOREN, DIE IN CHIRURGISCHEN WERKZEUGEN VERWENDET WERDEN
COUTEAUX POUR EFFECTEURS TERMINAUX UTILISÉS DANS LES OUTILS CHIRURGICAUX

(30) Priority: 06.03.2023 US 202318179168
(43) Date of publication of application: 26.03.2025
(73) Proprietor: Cilag GmbH International, 6300 Zug (CH)
(72) Inventor: FISCHER, Austin Michael, Blue Ash, Ohio 45242 (US); ZHANG, Guowei John, Blue Ash, Ohio 45242 (US); KOLPITCKE, Andrew, Blue Ash, Ohio 45242 (US); HSU, Wei-Liang, Blue Ash, Ohio 45242 (US); CHIANG, Pei-Lieh, Blue Ash, Ohio 45242 (US)
(74) Representative: Carpmaels & Ransford LLP
(86) International application number: PCT/IB2024/052059
(87) International publication number: WO 2024/184786

(56) References cited:
- DE-T2- 60 226 015
- DE-T2- 60 307 820
- US-A- 5 597 107
- US-A1- 2015 272 606
- US-A1- 2019 216 561
- US-A1- 2020 129 198
- US-A1- 2020 178 994

## Description

### BACKGROUND

Minimally invasive surgical (MIS) instruments are often preferred over traditional open surgical devices due to reduced post-operative recovery time and minimal scarring. Laparoscopic surgery is one type of MIS procedure in which one or more small incisions are formed in the abdomen of a patient and a trocar is inserted through the incision to form a pathway that provides access to the abdominal cavity. Through the trocar, a variety of instruments and surgical tools can be introduced into the abdominal cavity. The instruments and tools introduced into the abdominal cavity via the trocar can be used to engage and/or treat tissue in a number of ways to achieve a diagnostic or therapeutic effect.

Various robotic systems have been developed to assist in MIS procedures. Robotic systems can allow for more instinctive hand movements by maintaining natural eye-hand axis. Robotic systems can also allow for more degrees of freedom in movement by including an articulable "wrist" joint that creates a more natural hand-like articulation. In such systems, an end effector positioned at the distal end of the instrument can be articulated (moved) using a cable driven motion system having one or more drive cables that extend through the wrist joint. A user (e.g., a surgeon) is able to remotely operate the end effector by grasping and manipulating in space one or more controllers that communicate with a tool driver coupled to the surgical instrument. User inputs are processed by a computer system incorporated into the robotic surgical system, and the tool driver responds by actuating the cable driven motion system. Moving the drive cables articulates the end effector to desired angular positions and configurations.

Some end effectors also include a knife that is able to be advanced and retracted between opposing jaws to cut or sever tissue grasped between the opposing jaws. Improvements to the design and function of the knife are desirable to improve the efficiency of the end effector and any procedures undertaken with the end effector.

DE60226015T2 describes an electrosurgical instrument that includes a housing having a shaft attached thereto which connects a pair of first and second jaw members such that the jaw members are movable relative to one another to grasp tissue therebetween. The instrument also includes a four-bar handle assembly attached to the housing for actuating a drive rod assembly. The handle assembly includes a handle and a cam-like piston which cooperate to impart a uniform closure pressure against tissue grasped between the jaw members. The instrument also includes a rotating assembly for rotating the jaw members, a knife assembly for separating tissue and a pair of electrical leads connect the jaw members to a source of electrical energy. The electrical leads include slack loops disposed in the rotating assembly which permit rotation of the jaw members about the axis.

US2020178994A1 describes an electrosurgical instrument that includes first and second jaw members each including an outer jaw housing, a tissue-treating plate, and a longitudinally-extending knife channel defined therethrough. The electrosurgical instrument also includes a knife actuator and a knife assembly operably coupled to the knife actuator. The knife actuator is configured to advance at least a portion of the knife assembly through the knife channel to cut tissue disposed between the jaw members. The knife assembly includes an elongated shaft having a proximal portion coupled to the knife actuator and a distal portion defining at least one aperture therethrough. The knife assembly also includes a knife blade having a sharpened distal end configured to cut tissue and at least one raised portion extending through the aperture defined by the elongated shaft.

US5597107A describes a surgical stapler instrument for applying lateral lines of staples to tissue while cutting the tissue between those staple lines. The instrument includes a handle portion, an implement portion, a reciprocating section, a drive member and a movable actuator. The implement portion includes a staple cartridge and an anvil. The reciprocating section is adapted to move back and forth along an axis of the implement portion. The movable actuator is associated with the handle portion and is engaged with the drive member such that motion of the actuator causes the drive member to move back and forth between first and second drive positions separated by a first distance.

US2020129198A1 describes an end effector that includes first and second jaws movable between open and closed positions, a guide track defined in the second jaw, and a cutting element extendable into the guide track and longitudinally movable within the guide track. A retention feature is positioned within the guide track and operatively couples the cutting element to a drive rod. The drive rod is actuatable to move the cutting element within the guide track, and the retention feature is larger than a width of an opening to the guide track such that the retention feature is retained within the guide track as the cutting element moves.

DE60307820T2 describes an electrosurgical stapling apparatus that uses thermogenic energy as well as surgical fasteners or staples for strengthening tissue, providing hemostasis, tissue joining or welding. The thermogenic energy also strengthens tissue in proximity to a staple line and knife cut line and provides hemostasis along the staple and cut lines formed by the staples and a knife blade during surgical stapling. The use thermogenic energy provides short-term hemostasis and sealing, and reduces or prevents staple line and cut line bleeding, while the stapling features provide short and long-term tissue strength and hemostasis. The stapling apparatus further substantially reduces or prevents knife cut line bleeding by energizing a knife blade for cauterizing tissue while it is being cut. In one embodiment, energy is applied to the anvil to energize the staples as they make contact with the anvil.

### BRIEF DESCRIPTION OF THE DRAWINGS

The following figures are included to illustrate certain aspects of the present disclosure, and should not be viewed as exclusive embodiments. The subject matter disclosed is capable of considerable modifications, alterations, combinations, and equivalents in form and function, without departing from the scope of this disclosure. The scope of the invention is defined by claim 1.
FIG. 1 is a block diagram of an example robotic surgical system that may incorporate some or all of the principles of the present disclosure.
FIG. 2 is an isometric side view of an example surgical tool that may incorporate some or all of the principles of the present disclosure.
FIG. 3 illustrates potential degrees of freedom in which the wrist of the surgical tool of FIG. 2 may be able to articulate (pivot) and translate.
FIG. 4 is an enlarged isometric view of the distal end of the surgical tool of FIG. 2.
FIG. 5 is another enlarged isometric view of the distal end of the surgical tool of FIG. 2, according to one or more embodiments of the present disclosure.
FIGS. 6A and 6B are enlarged isometric views of the knife and the knife housing of FIG. 5, according to one or more embodiments.
FIGS. 7A and 7B are side and exploded isometric views, respectively, of the knife and the distal end of the drive rod, according to one or more embodiments.
FIGS. 8A and 8B are side and exploded isometric views, respectively, of another example of the knife as coupled to the distal end of the drive rod, according to one or more additional embodiments.
FIG. 9 is an enlarged side view of the end effector with the knife during firing, according to one or more embodiments.

### DETAILED DESCRIPTION

The present disclosure is related to surgical tools and, more particularly, to end effectors having improved knife attachment and functionality.

Embodiments discussed herein describe an end effector for a surgical tool, where the end effector includes opposing first and second jaws, and a knife slot defined in one or both of the first and second jaws. A knife is extendable through the knife slot and may be operatively coupled to a knife rod at a retention feature secured to a distal end of the drive rod. More specifically, a central notch may be defined in the retention feature, and the knife may define a locking feature receivable within the central notch to axially constrain the knife to the retention feature and the knife rod. In at least one embodiment, the central notch extends into a material of the drive rod. Securing the knife to the drive rod at the locking feature and the central notch may prove advantageous in transmitting axial loads from the knife to the drive rod, and thus helping to prevent premature failure of the knife.

In some embodiments, a blade is provided at a leading end of the knife and includes a leading cutting edge extending perpendicular to a sealing plane provided between the opposing first and second jaws when the opposing first and second jaws are closed, and an angled cutting edge extending from the leading cutting edge at a transition point. The angled cutting edge extends from the leading cutting edge at an angle offset from perpendicular to the sealing plane. Having the leading cutting edge perpendicular to the sealing plane may provide a longer stroke length for the knife.

In some embodiments, the trailing end of the knife may provide a reduced trailing profile defined by a first arcuate surface extending from a top of the knife and transitioning to a second arcuate surface that transitions to a third arcuate surface. The reduced trailing profile may prove advantageous as the knife traverses the knife slot, which may be curved. While other knives without the reduced trailing profile may catch or contact portions of the curved knife slot, the reduced trailing profile eliminates this friction generating inefficiency.

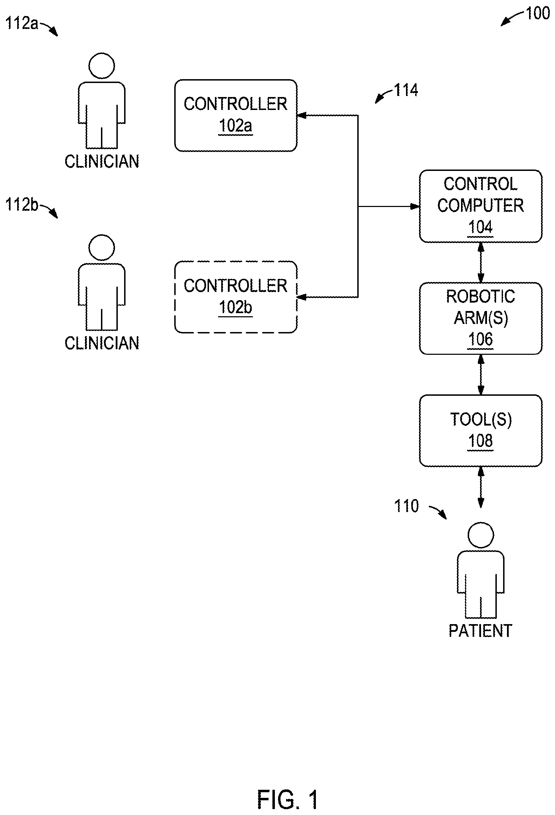

FIG. 1 is a block diagram of an example robotic surgical system 100 that may incorporate some or all of the principles of the present disclosure. As illustrated, the system 100 can include at least one set of user input controllers 102a and at least one control computer 104. The control computer 104 may be mechanically and/or electrically coupled to a robotic manipulator and, more particularly, to one or more robotic arms 106 (alternately referred to as "tool drivers"). In some embodiments, the robotic manipulator may be included in or otherwise mounted to an arm cart capable of making the system portable. Each robotic arm 106 may include and otherwise provide a location for mounting one or more surgical instruments or tools 108 for performing various surgical tasks on a patient 110. Operation of the robotic arms 106 and associated tools 108 may be directed by a clinician 112a (e.g., a surgeon) from the user input controller 102a.

In some embodiments, a second set of user input controllers 102b (shown in dashed line) may be operated by a second clinician 112b to direct operation of the robotic arms 106 and tools 108 via the control computer 104 and in conjunction with the first clinician 112a. In such embodiments, for example, each clinician 112a,b may control different robotic arms 106 or, in some cases, complete control of the robotic arms 106 may be passed between the clinicians 112a,b as needed. In some embodiments, additional robotic manipulators having additional robotic arms may be utilized during surgery on the patient 110, and these additional robotic arms may be controlled by one or more of the user input controllers 102a,b.

The control computer 104 and the user input controllers 102a,b may be in communication with one another via a communications link 114, which may be any type of wired or wireless telecommunications means configured to carry a variety of communication signals (e.g., electrical, optical, infrared, etc.) according to any communications protocol. In some applications, for example, there is a tower with ancillary equipment and processing cores designed to drive the robotic arms 106.

The user input controllers 102a,b generally include one or more physical controllers that can be grasped by the clinicians 112a,b and manipulated in space while the surgeon views the procedure via a stereo display. The physical controllers generally comprise manual input devices movable in multiple degrees of freedom, and which often include an actuatable handle for actuating the surgical tool(s) 108, for example, for opening and closing opposing jaws, applying an electrical potential (current) to an electrode, or the like. The control computer 104 can also include an optional feedback meter viewable by the clinicians 112a,b via a display to provide a visual indication of various surgical instrument metrics, such as the amount of force being applied to the surgical instrument (i.e., a cutting instrument or dynamic clamping member).

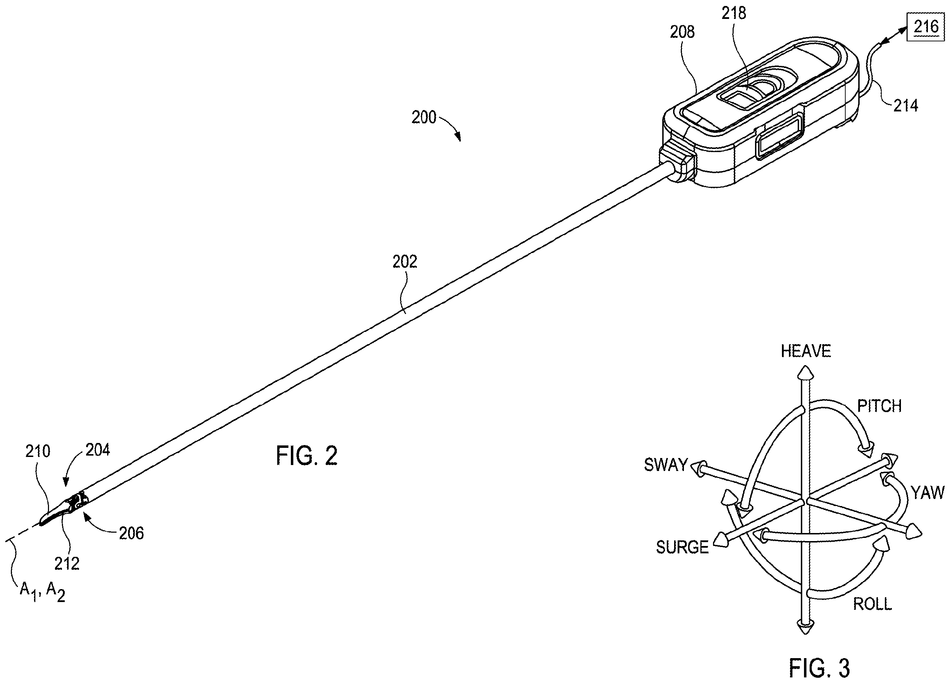

FIG. 2 is an isometric side view of an example surgical tool 200 that may incorporate some or all of the principles of the present disclosure. The surgical tool 200 may be the same as or similar to the surgical tool(s) 108 of FIG. 1 and, therefore, may be used in conjunction with a robotic surgical system, such as the robotic surgical system 100 of FIG. 1. Accordingly, the surgical tool 200 may be designed to be releasably coupled to a tool driver included in the robotic surgical system 100. In other embodiments, however, aspects of the surgical tool 200 may be adapted for use in a manual or hand-operated manner, without departing from the scope of the disclosure.

As illustrated, the surgical tool 200 includes an elongated shaft 202, an end effector 204, a wrist 206 (alternately referred to as a "wrist joint" or an "articulable wrist joint") that couples the end effector 204 to the distal end of the shaft 202, and a drive housing 208 coupled to the proximal end of the shaft 202. In applications where the surgical tool is used in conjunction with a robotic surgical system (e.g., the robotic surgical system 100 of FIG. 1), the drive housing 208 can include coupling features that releasably couple the surgical tool 200 to the robotic surgical system.

The terms "proximal" and "distal" are defined herein relative to a robotic surgical system having an interface configured to mechanically and electrically couple the surgical tool 200 (e.g., the housing 208) to a robotic manipulator. The term "proximal" refers to the position of an element closer to the robotic manipulator and the term "distal" refers to the position of an element closer to the end effector 204 and thus further away from the robotic manipulator. Alternatively, in manual or hand-operated applications, the terms "proximal" and "distal" are defined herein relative to a user, such as a surgeon or clinician. The term "proximal" refers to the position of an element closer to the user and the term "distal" refers to the position of an element closer to the end effector 204 and thus further away from the user. Moreover, the use of directional terms such as above, below, upper, lower, upward, downward, left, right, and the like are used in relation to the illustrative embodiments as they are depicted in the figures, the upward or upper direction being toward the top of the corresponding figure and the downward or lower direction being toward the bottom of the corresponding figure.

During use of the surgical tool 200, the end effector 204 is configured to move (pivot) relative to the shaft 202 at the wrist 206 to position the end effector 204 at desired orientations and locations relative to a surgical site. To accomplish this, the housing 208 includes (contains) various drive inputs and mechanisms (e.g., gears, actuators, etc.) designed to control operation of various features associated with the end effector 204 (e.g., clamping, firing, cutting, rotation, articulation, etc.). In at least some embodiments, the shaft 202, and hence the end effector 204 coupled thereto, is configured to rotate about a longitudinal axis A₁ of the shaft 202. In such embodiments, at least one of the drive inputs included in the housing 208 is configured to control rotational movement of the shaft 202 about the longitudinal axis A₁.

The shaft 202 is an elongate member extending distally from the housing 208 and has at least one lumen extending therethrough along its axial length. In some embodiments, the shaft 202 may be fixed to the housing 208, but could alternatively be rotatably mounted to the housing 208 to allow the shaft 202 to rotate about the longitudinal axis A₁. In yet other embodiments, the shaft 202 may be releasably coupled to the housing 208, which may allow a single housing 208 to be adaptable to various shafts having different end effectors.

The end effector 204 can exhibit a variety of sizes, shapes, and configurations. In the illustrated embodiment, the end effector 204 comprises a combination tissue grasper and vessel sealer that include opposing first (upper) and second (lower) jaws 210, 212 configured to move (articulate) between open and closed positions. As will be appreciated, however, the opposing jaws 210, 212 may alternatively form part of other types of end effectors such as, but not limited to, a surgical scissors, a clip applier, a needle driver, a babcock including a pair of opposed grasping jaws, bipolar jaws (e.g., bipolar Maryland grasper, forceps, a fenestrated grasper, etc.), etc. One or both of the jaws 210, 212 may be configured to pivot to articulate the end effector 204 between the open and closed positions.

FIG. 3 illustrates the potential degrees of freedom in which the wrist 206 may be able to articulate (pivot) and thereby move the end effector 204. The wrist 206 can have any of a variety of configurations. In general, the wrist 206 comprises a joint configured to allow pivoting movement of the end effector 204 relative to the shaft 202. The degrees of freedom of the wrist 206 are represented by three translational variables (i.e., surge, heave, and sway), and by three rotational variables (i.e., Euler angles or roll, pitch, and yaw). The translational and rotational variables describe the position and orientation of the end effector 204 with respect to a given reference Cartesian frame. As depicted in FIG. 3, "surge" refers to forward and backward translational movement, "heave" refers to translational movement up and down, and "sway" refers to translational movement left and right. With regard to the rotational terms, "roll" refers to tilting side to side, "pitch" refers to tilting forward and backward, and "yaw" refers to turning left and right.

The pivoting motion can include pitch movement about a first axis of the wrist 206 (e.g., X-axis), yaw movement about a second axis of the wrist 206 (e.g., Y-axis), and combinations thereof to allow for 360° rotational movement of the end effector 204 about the wrist 206. In other applications, the pivoting motion can be limited to movement in a single plane, e.g., only pitch movement about the first axis of the wrist 206 or only yaw movement about the second axis of the wrist 206, such that the end effector 204 moves only in a single plane.

Referring again to FIG. 2, the surgical tool 200 may also include a plurality of drive cables (obscured in FIG. 2) that form part of a cable driven motion system configured to facilitate actuation and articulation of the end effector 204 relative to the shaft 202. Moving (actuating) one or more of the drive cables moves the end effector 204 between an unarticulated position and an articulated position. The end effector 204 is depicted in FIG. 2 in the unarticulated position where a longitudinal axis A₂ of the end effector 204 is substantially aligned with the longitudinal axis A₁ of the shaft 202, such that the end effector 204 is at a substantially zero angle relative to the shaft 202. Due to factors such as manufacturing tolerance and precision of measurement devices, the end effector 204 may not be at a precise zero angle relative to the shaft 202 in the unarticulated position, but nevertheless be considered "substantially aligned" thereto. In the articulated position, the longitudinal axes A₁, A₂ would be angularly offset from each other such that the end effector 204 is at a non-zero angle relative to the shaft 202.

In some embodiments, the surgical tool 200 may be supplied with electrical power (current) via a power cable 214 coupled to the housing 208. In other embodiments, the power cable 214 may be omitted and electrical power may be supplied to the surgical tool 200 via an internal power source, such as one or more batteries, capacitors, or fuel cells. In such embodiments, the surgical tool 200 may alternatively be characterized and otherwise referred to as an "electrosurgical instrument" capable of providing electrical energy to the end effector 204.

The power cable 214 may place the surgical tool 200 in electrical communication with a generator 216 that supplies energy, such as electrical energy (e.g., radio frequency energy), ultrasonic energy, microwave energy, heat energy, or any combination thereof, to the surgical tool 200 and, more particularly, to the end effector 204. Accordingly, the generator 216 may comprise a radio frequency (RF) source, an ultrasonic source, a direct current source, and/or any other suitable type of electrical energy source that may be activated independently or simultaneously.

In applications where the surgical tool 200 is configured for bipolar operation, the power cable 214 will include a supply conductor and a return conductor. Current can be supplied from the generator 216 to an active (or source) electrode located at the end effector 204 via the supply conductor, and current can flow back to the generator 216 via a return electrode located at the end effector 204 via the return conductor. In the case of a bipolar grasper with opposing jaws, for example, the jaws serve as the electrodes where the proximal end of the jaws are isolated from one another and the inner surface of the jaws (i.e., the area of the jaws that grasp tissue) apply the current in a controlled path through the tissue. In applications where the surgical tool 200 is configured for monopolar operation, the generator 216 transmits current through a supply conductor to an active electrode located at the end effector 204, and current is returned (dissipated) through a return electrode (e.g., a grounding pad) separately coupled to a patient's body.

The surgical tool 200 may further include a manual release switch 218 that may be manually actuated by a user (e.g., a surgeon) to open the jaws 210, 212. The release switch 218 is movably positioned on the drive housing 208, and a user is able to manually move (slide) the release switch 218 from a disengaged position, as shown, to an engaged position. In the disengaged position, the surgical tool 200 is able to operate as normal. As the release switch 218 moves to the engaged position, however, various internal component parts of the drive housing 208 are simultaneously moved, thereby resulting in the jaws 210, 212 opening, which might prove beneficial for a variety of reasons. In some applications, for example, the release switch 218 may be moved in the event of an electrical disruption that renders the surgical tool 200 inoperable. In such applications, the user would be able to manually open the jaws 210, 212 and thereby release any grasped tissue and remove the surgical tool 200. In other applications, the release switch 218 may be actuated (enabled) to open the jaws 210, 212 in preparation for cleaning and/or sterilization of the surgical tool 200. In some applications, the surgical tool 200 is first decoupled from the robotic manipulator and the associated motors, following which the user can actuate the manual release switch 218 to move the associated inputs and drive the cables once the motors are disengaged.

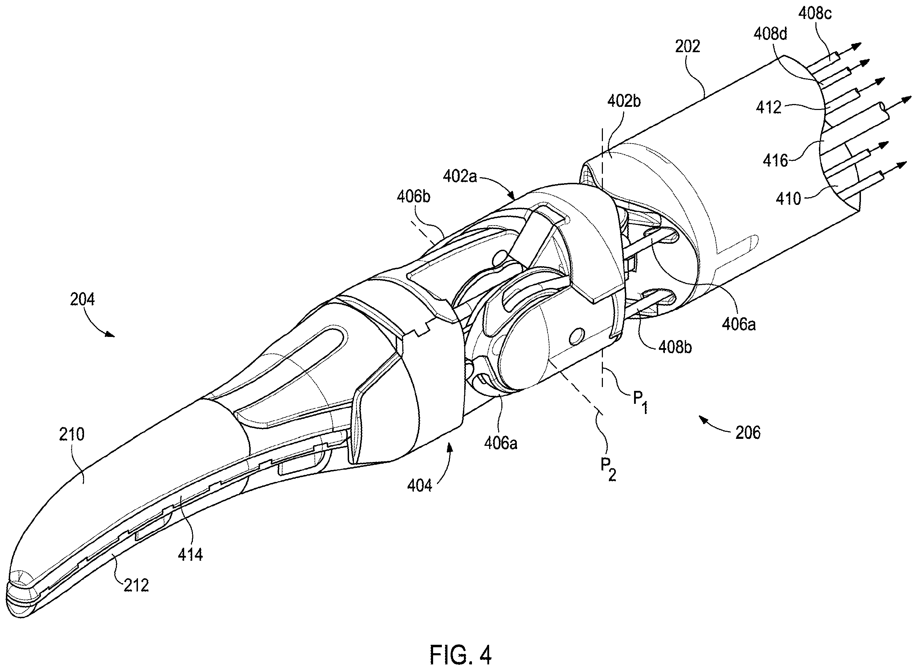

FIG. 4 is an enlarged isometric view of the distal end of the surgical tool 200. More specifically, FIG. 4 depicts an enlarged view of the end effector 204 and the wrist 206, with the jaws 210, 212 of the end effector 204 in the closed position. The wrist 206 operatively couples the end effector 204 to the shaft 202. In some embodiments, however, a shaft adapter may be directly coupled to the wrist 206 and otherwise interpose the shaft 202 and the wrist 206. Accordingly, the wrist 206 may be operatively coupled to the shaft 202 either through a direct coupling engagement where the wrist 206 is directly coupled to the distal end of the shaft 202, or an indirect coupling engagement where a shaft adapter interposes the wrist 206 and the distal end of the shaft 202. As used herein, the term "operatively couple" refers to a direct or indirect coupling engagement between two components.

To operatively couple the end effector 204 to the shaft 202, the wrist 206 includes a first or "distal" clevis 402a and a second or "proximal" clevis 402b. The clevises 402a,b are alternatively referred to as "articulation joints" of the wrist 206 and extend from the shaft 202, or alternatively a shaft adapter. The clevises 402a,b are operatively coupled to facilitate articulation of the wrist 206 relative to the shaft 202. As illustrated, the wrist 206 also includes a linkage 404 arranged distal to the distal clevis 402a and operatively mounted to the jaws 210, 212.

As illustrated, the proximal end of the distal clevis 402a may be rotatably mounted or pivotably coupled to the proximal clevis 402b at a first pivot axis P₁ of the wrist 206. In some embodiments, an axle may extend through the first pivot axis P₁ and the distal and proximal clevises 402a,b may be rotatably coupled via the axle. In other embodiments, however, such as is depicted in FIG. 4, the distal and proximal clevises 402a,b may be engaged in rolling contact, such as via an intermeshed gear relationship that allows the clevises 402a,b to rotate relative to each other similar to a rolling joint.

First and second pulleys 406a and 406b may be rotatably mounted to the distal end of the distal clevis 402a at a second pivot axis P₂ of the wrist 206. The linkage 404 may be arranged distal to the second pivot axis P₂ and operatively mounted to the jaws 210, 212. The first pivot axis P₁ is substantially perpendicular (orthogonal) to the longitudinal axis A₁ of the shaft 202, and the second pivot axis P₂ is substantially perpendicular (orthogonal) to both the longitudinal axis A₁ and the first pivot axis P₁. Movement of the end effector 204 about the first pivot axis P₁ provides "yaw" articulation of the wrist 206, and movement about the second pivot axis P₂ provides "pitch" articulation of the wrist 206.

A plurality of drive cables, shown as drive cables 408a, 408b, 408c, and 408d, extend longitudinally within a lumen 410 defined by the shaft 202 (or a shaft adaptor) and extend at least partially through the wrist 206. The drive cables 408a-d may form part of the cable driven motion system housed within the drive housing 208 (FIG. 2), and may comprise cables, bands, lines, cords, wires, woven wires, ropes, strings, twisted strings, elongate members, belts, shafts, flexible shafts, drive rods, or any combination thereof. The drive cables 408a-d can be made from a variety of materials including, but not limited to, a metal (e.g., tungsten, stainless steel, nitinol, etc.), a polymer (e.g., ultra-high molecular weight polyethylene), a synthetic fiber (e.g., KEVLAR^{®}, VECTRAN^{®}, etc.), an elastomer, or any combination thereof. While four drive cables 408a-d are depicted in FIG. 4, more or less than four may be employed, without departing from the scope of the disclosure.

The drive cables 408a-d extend proximally from the end effector 204 and the wrist 206 toward the drive housing 208 (FIG. 2) where they are operatively coupled to various actuation mechanisms or devices that facilitate longitudinal movement (translation) of the drive cables 408a-d within the lumen 410. Selective actuation of the drive cables 408a-d applies tension (i.e., pull force) to the given drive cable 408a-d in the proximal direction, which urges the given drive cable 408a-d to translate longitudinally within the lumen 410.

In the illustrated embodiment, the drive cables 408a-d each extend longitudinally through the proximal clevis 402b. The distal end of each drive cable 408a-d terminates at the first or second pulleys 406a,b, thus operatively coupling each drive cable 408a-d to the end effector 204. In some embodiments, the distal ends of the first and second drive cables 408a,b may be coupled to each other and terminate at the first pulley 406a, and the distal ends of the third and fourth drive cables 408c,d may be coupled to each other and terminate at the second pulley 406b. In at least one embodiment, the distal ends of the first and second drive cables 408a,b and the distal ends of the third and fourth drive cables 408c,d may each be coupled together at corresponding ball crimps (not shown) mounted to the first and second pulleys 406a,b, respectively.

In at least one embodiment, the drive cables 408a-d may operate "antagonistically". More specifically, when the first drive cable 408a is actuated (moved), the second drive cable 408b naturally follows as coupled to the first drive cable 408a, and when the third drive cable 408c is actuated, the fourth drive cable 408d naturally follows as coupled to the third drive cable 408c, and vice versa. Antagonistic operation of the drive cables 408a-d can open or close the jaws 210, 212 and can further cause the end effector 204 to articulate at the wrist 206. More specifically, selective actuation of the drive cables 408a-d in known configurations or coordination can cause the end effector 204 to articulate about one or both of the pivot axes P₁, P₂, thus facilitating articulation of the end effector 204 in both pitch and yaw directions. Moreover, selective actuation of the drive cables 408a-d in other known configurations or coordination will cause the jaws 210, 212 to open or close. Antagonistic operation of the drive cables 408a-d advantageously reduces the number of cables required to provide full wrist 206 motion, and also helps eliminate slack in the drive cables 408a-d, which results in more precise motion of the end effector 204.

In the illustrated embodiment, the end effector 204 is able to articulate (move) in pitch about the second or "pitch" pivot axis P₂, which is located near the distal end of the wrist 206. Thus, the jaws 210, 212 open and close in the direction of pitch. In other embodiments, however, the wrist 206 may alternatively be configured such that the second pivot axis P₂ facilitates yaw articulation of the jaws 210, 212, without departing from the scope of the disclosure.

In some embodiments, an electrical conductor 412 may also extend longitudinally within the lumen 410, through the wrist 206, and terminate at an electrode 414 to supply electrical energy to the end effector 204. In some embodiments, the electrical conductor 412 may comprise a wire, but may alternatively comprise a rigid or semi-rigid shaft, rod, or strip (ribbon) made of a conductive material. The electrical conductor 412 may be entirely or partially covered with an insulative covering (overmold) made of a non-conductive material. Using the electrical conductor 412 and the electrode 414, the end effector 204 may be configured for monopolar or bipolar RF operation.

In the illustrated embodiment, the end effector 204 comprises a combination tissue grasper and vessel sealer that includes a knife (not visible), alternately referred to as a "cutting element" or "blade." The knife is aligned with and configured to traverse a guide track or "knife slot" (not visible) defined longitudinally in one or both of the upper and lower jaws 210, 212. The knife may be operatively coupled to the distal end of a drive rod 416 (alternately referred to as "knife rod," "actuation rod," or "push rod") that extends longitudinally within the lumen 410 and passes through the wrist 206. Longitudinal movement (translation) of the drive rod 416 correspondingly moves the knife within the knife slot(s). Similar to the drive cables 408a-d, the drive rod 416 may form part of the actuation systems housed within the drive housing 208 (FIG. 2). Selective actuation of a corresponding drive input will cause the drive rod 416 to move distally or proximally within the lumen 410, and correspondingly move the knife in the same longitudinal direction.

The drive rod 416 may comprise a rigid or semi rigid elongate member, such as a rod or shaft (e.g., a hypotube, a hollow rod, a solid rod, etc.), a wire, a ribbon, a push cable, or any combination thereof. The drive rod 416 can be made from a variety of materials including, but not limited to, metal (e.g., tungsten, nitinol, stainless steel, etc.), a polymer, or a composite material. The drive rod 416 may have a circular cross-section, but may alternatively exhibit a polygonal cross-section without departing from the scope of the disclosure.

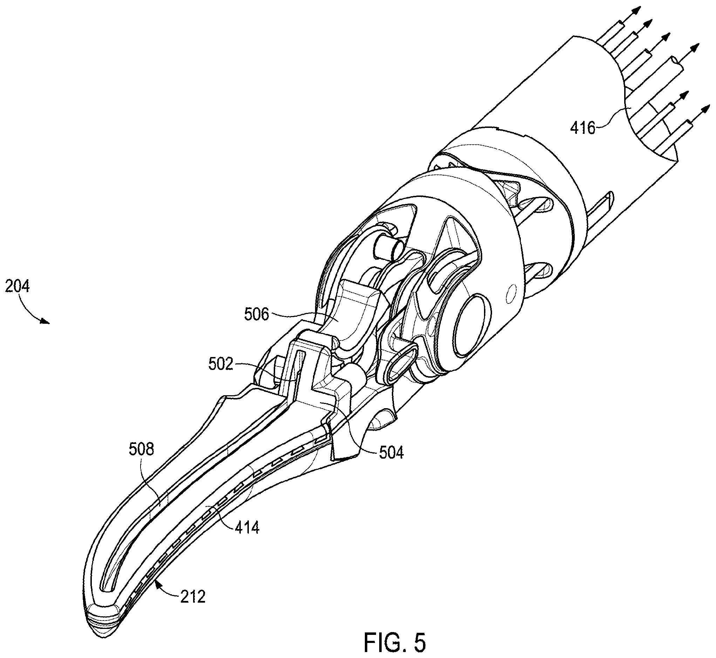

FIG. 5 is another enlarged isometric view of the end effector 204, according to one or more embodiments of the present disclosure. The upper jaw 210 (FIGS. 2 and 4) is omitted from FIG. 5 to enable viewing of various internal features of the end effector 204.

In the illustrated embodiment, a knife 502 (mostly occluded) is shown received within a portion of the electrode 414 of the lower jaw 212 and, more particularly, within a portion of an insulator 504 coupled to the electrode 414. In its retracted position, as shown in FIG. 5, the knife 502 may also be partially received within a knife housing 506 mounted to the end effector 204 between the upper and lower jaws 210, 212. The lower jaw 212 provides or otherwise defines a knife slot 508 through which the knife 502 may traverse upon distal actuation of the drive rod 416. While the knife slot 508 is shown provided in the lower jaw 212, in some embodiments, the knife slot 508 may be cooperatively defined by both the upper and lower jaws 210, 212.

As described in more detail below, the knife housing 506 defines a central passageway through which the drive rod 416 is able to extend to move the knife 502 along the knife slot 508. Upon firing the end effector 204, the drive rod 416 is moved (urged) distally, which correspondingly moves the knife 502 out of the knife housing 506 and into the knife slot 508. After firing is complete, the drive rod 416 is retracted proximally, which pulls the knife 502 proximally and back into the knife housing 506 until it is desired to again fire the end effector 204.

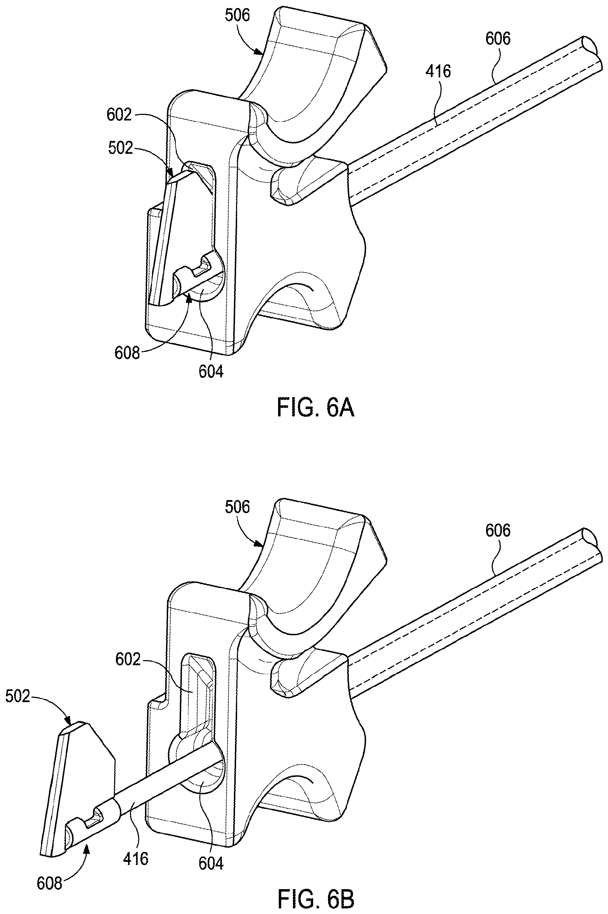

FIGS. 6A and 6B are enlarged isometric views of the knife 502 and the knife housing 506, according to one or more embodiments. In FIG. 6A, the knife 502 is shown in a first or "stowed" position, where the knife 502 is at least partially received within a cavity 602 defined by the knife housing 506 and sized to receive and "stow" the knife 502 when not in use. In FIG. 6B, the knife 502 is shown in a second or "extended" position, where the knife 502 is extended distally out of the cavity 602.

As mentioned above, the knife 502 may be operatively coupled to the distal end of the drive rod 416 (shown in dashed lines in FIG. 6A). A central passageway 604 is defined through the knife housing 506 and provides a conduit through which the drive rod 416 is able to extend to move the knife 502 into and along the knife slot 508 (FIG. 5). In at least one embodiment, the cavity 602 may form part of or communicate with the central passageway 604. In some embodiments, the drive rod 416 may comprise a solid shaft, but may alternatively comprise a tube or tubular structure. Moreover, the drive rod 416 may be made of a variety of flexible materials including, but not limited to, a metal or metal alloy (e.g., a nickel-titanium alloy or "nitinol"), a plastic or thermoplastic material, a composite material, or any combination thereof. The drive rod 416 may also comprise a braided cable construction of a metal (e.g., stainless steel, tungsten, etc.), or any of the aforementioned materials, and such braided cable may be radially constrained to support axial loads.

In some embodiments, as illustrated, a flexible sheath 606 (e.g., a hypotube or the like) may cover at least a portion of the drive rod 416. The sheath 606 may support and help prevent buckling of the drive rod 416 when assuming compressive loads during articulation of the wrist 206 (FIGS. 2 and 4) and opening and closure of the jaws 210, 212 (FIGS. 2 and 4). Similar to the drive rod 416, the flexible sheath 606 may be made of a variety of flexible materials including, but not limited to, a metal or metal alloy (e.g., a nickel-titanium alloy or "nitinol"), a metallic coil, a plastic or thermoplastic material, a composite material, a braided tubular, or any combination thereof.

The knife 502 may be attached to the distal end of the drive rod 416 at a connecting or "retention" feature 608. The retention feature 608 may comprise any attachment or coupling means that removably or permanently fixes the knife 502 to the drive rod 416. For example, the retention feature 608 may comprise, but is not limited to, a crimped engagement, a welded interface, an adhesive attachment, an interference or shrink fit, an overmold (e.g., a shaped block of material or a support block), one or more mechanical fasteners, or any combination thereof. In at least one embodiment, the retention feature 608 may comprise a metal tube crimped or press fit to the distal end of the drive rod 416.

Upon firing the end effector 204 (FIGS. 2 and 4), the drive rod 416 is moved (urged) distally through the central passageway 604, which correspondingly moves the knife 502 to the extended position and otherwise out of the cavity 602 and into the knife slot 508 (FIG. 5). As the drive rod 416 translates distally, the sheath 606 supports the drive rod 416 against axial buckling resulting from compressive loading on the drive rod 416. After firing is complete, the drive rod 416 is retracted proximally, which correspondingly pulls the knife 502 proximally and back to the stowed position and otherwise into the cavity 602 until it is desired to again fire the end effector 204.

Some end effectors, such as the end effector 204 of FIGS. 2 and 4, can articulate in two planes simultaneously, which requires the knife 502 and the drive rod 416 to traverse the articulation joints while still applying adequate cut force. Moreover, moving knife 502 in the retract direction may result in contacting a hard stop, retract overdrive prevention, or positional locator to be fed into manufacturing settings or controls schemes. In some cases, this can cause the knife 502 to prematurely fail and otherwise separate from the retention feature 608 and/or the drive rod 416. According to embodiments of the present disclosure, and as described in more detail below, the knife 502 may be operatively coupled to the retention feature 608 and the drive rod 416 with a mechanical locking feature, which helps assume any axial loading resulting, including retract, retraction overdrive prevention, and positional locating loadings.

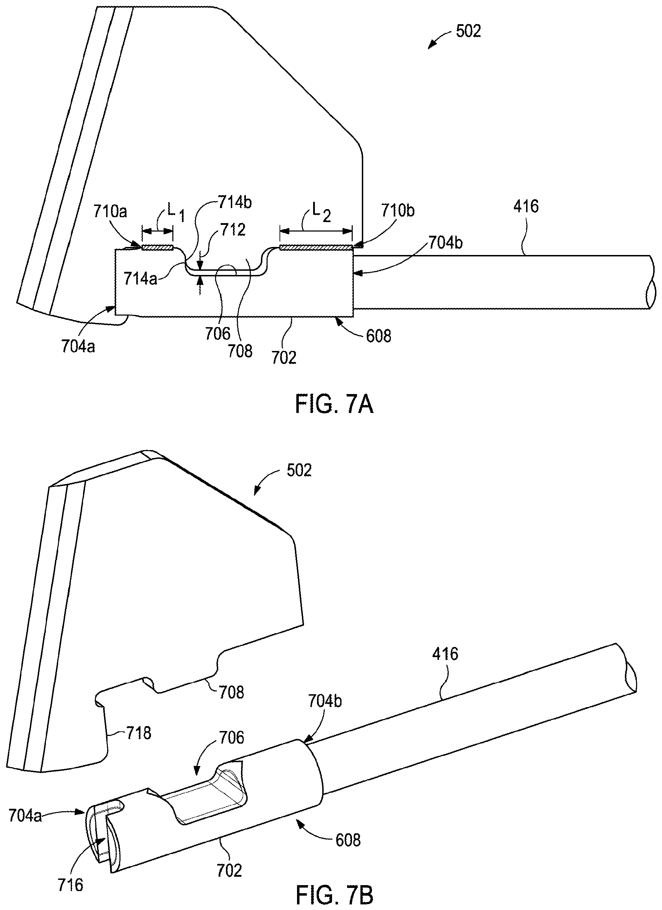

FIGS. 7A and 7B are side and exploded isometric views, respectively, of the knife 502 as coupled to the distal end of the drive rod 416, according to one or more embodiments. As illustrated, the retention feature 608 may be secured to the distal end of the drive rod 416. The retention feature 608 may be made of a material capable of allowing the knife 502 to be welded thereto. In at least one embodiment, for example, the retention feature 608 may be made of a metal, such as stainless steel. In embodiments where the knife 502 is made of Nitinol, the retention feature 608 may also be made of Nitinol.

As illustrated, the retention feature 608 may comprise a short tubular length or tube. More specifically, the retention feature 608 may comprise a tubular body 702 having a first or "distal" and 704a and a second or "proximal" end 704b opposite the distal end 704a. The tubular body 702 may provide an inner conduit or passageway sized to receive the distal end of the drive rod 416a. As mentioned above, the body 702 of the retention feature 608 may be secured to the drive rod 416 in a variety of ways including, but not limited to, crimping, press fitting, shrink fitting, an adhesive, or any combination thereof.

Once the retention feature 608 is secured to the distal end of the drive rod 416, a central notch 706 may be cut into and otherwise defined in the body 702 at a location between the distal and proximal ends 704a,b. As illustrated, the central notch 706 may extend through the side wall of the body 702 and partially into the underlying material of the drive rod 416. In some embodiments, the central notch 706 may be defined and otherwise formed via grinding or through a grinding process, but could alternatively be formed through other processes, such as laser cutting, wire electrical discharge machining (EDM), milling, chemical etching, water jetting, stamping, or the like. In some embodiments, the depth of the central notch 706 into the material of the drive rod 416 may stop short of the centerline of the drive rod 416. In other embodiments, however the depth of the central notch 706 may extend past the centerline of the drive rod 416, without departing from the scope of the disclosure.

In some embodiments, as illustrated, the knife 502 may provide and otherwise define a locking feature 708 sized or configured to be received within the central notch 706 when the knife 502 is properly mounted to the retention feature 608. In one or more embodiments, the locking feature 708 may be laser cut into the body of the knife 502, but could alternatively be defined via other manufacturing or forming processes, without departing from the scope of the disclosure.

In the illustrated embodiment, the locking feature 708 comprises a generally rectangular projection or tab that extends from the bottom of the main body of the knife 502. In other embodiments, however, the locking feature 708 may exhibit other geometric shapes including, but not limited to, angled, arcuate, rounded, or any combination thereof. In some embodiments, when mounting the knife 502 to the retention feature 608, the locking feature 708 may extend into the central notch 706 but not contact the bottom of the central notch 706, such as the material of the drive rod 416. In such embodiments, a gap 712 may be defined between the bottom of the central notch 706 and the outer extent of the locking feature 708. In other embodiments, however, the locking feature 708 may be designed and otherwise configured to contact or rest on the bottom of the central notch 706.

As best seen in FIG. 7A, once the locking feature 708 is received within the central notch 706, the knife 502 may be welded to the outer surface of the retention feature 608 at one or more locations. More specifically, the knife 502 may be welded to the retention feature 608 at a first weld 710a located distal to the central notch 706, and a second weld 710a located proximal to the central notch 706. In other embodiments, however, one of the welds 710a,b may be omitted, without departing from the scope of the disclosure. In some applications, the gap 712 may prove advantageous in ensuring that there is intimate (direct) contact between the retention feature 608 and the blade 502, which can promote more robust welds 710a,b.

In some embodiments, the length L₁ of the distal weld 710a may be smaller than the length L₂ of the proximal weld 710b. This may prove advantageous since the stress concentration region of the drive rod 416 (e.g., the proximal notch corner) is further away from where the drive rod 416 exits the retention feature 608 such that it takes less of the bending loading (from articulation and retraction) further away from the proximal edge. In addition, the retraction loads commonly result in the primary loading of the proximal-most region of the weld, so having a more robust (longer) proximal weld may help mitigate separation of the knife 502 from the retention feature 608. In other embodiments, however, the length L₁ of the distal weld 710a may be larger than the length L₂ of the proximal weld 710b, or the lengths L₁, L₂ may be the same, without departing from the scope of the disclosure.

In some embodiments, the length from the proximal end of the central notch 716 to the proximal end of the retention feature 608 may be longer than the length from the distal end of the central notch 706 to the distal end of the retention feature 608. This may help accommodate the longer length L₂ versus the shorter length L₁, but may also prove advantageous in helping with fatigue life as there is larger length between the bending flexible region of the knife rod 416 to the area of the central notch 706.

In at least one embodiment, the locking feature 708 may be received within the central notch 706 such that a distal end 714a of the locking feature 708 contacts and otherwise engages an opposing distal end 714b of the central notch 706, and thereby axially constrains the knife 502 to the retention features 608 and the drive rod 416. More particularly, engaging the opposing distal ends 714a,b may prove advantageous in transmitting axial loads from the knife 502 to the drive rod 416 and thus helping to prevent premature failure of the knife 502; e.g., separation of the knife 502 from the retention feature 608. More particularly, when the knife 502 is positionally located or retracted, as briefly mentioned above, the knife 502 is moved proximally and stowed within the knife housing 506 (FIGS. 6A-6B). During a retraction after firing or a positional locating sequence, an axial load is applied on the drive rod 416 to return the knife 502 and/or force the knife 502 into axial engagement with the knife housing 506. Having the opposing distal ends 714a,b in contact allows the knife 502 to transmit a resulting axial load caused by contact with the knife housing 506 directly to the drive rod 416. If the opposing distal ends 714a,b were not in contact, the resulting axial load applied to the knife 502 would be assumed by the welds 710a,b, which could result in the knife 502 separating from the retention feature 608 at the welds 710a,b.

Accordingly, while the welds 710a,b may help vertically constrain the knife 502 to the drive rod 416 via the retention feature 608, receiving the locking feature 708 within the central notch 706 may provide additional axial constraint and load transfer that helps prevent the knife 502 from prematurely separating from the drive rod 416. Consequently, the locking feature 708 enables sustaining of higher and increased retraction, retract over drive prevention, and positional locating loads.

As best seen in FIG. 7B, in some embodiments, an end notch 716 may be cut into and otherwise defined in the distal end of the drive rod 416. As illustrated, the end notch 716 may extend through the distal end 704a of the retention feature 608 and partially into the material of the drive rod 416. The end notch 716 may be defined and otherwise formed through any of the processes mentioned above for forming the central notch 706. In at least one embodiment, as illustrated, the end notch 716 may extend orthogonal to the central notch 706.

In some embodiments, as best seen in FIG. 7B, the knife 502 may provide and otherwise define an end tab 718 sized to be received within the end notch 716. Receiving the end tab 718 in the end notch 716 may help laterally stabilize the knife 502 when mounted to the retention feature 608. Moreover, receiving the end tab 718 in the end notch 716 may further prove advantageous in transferring axial loads in the fire direction directly from the drive rod 416 to the end tab 718. In at least one embodiment, the knife 502 may further be welded to the distal end of the drive rod 416 at the interface between the end tab and the end notch 716.

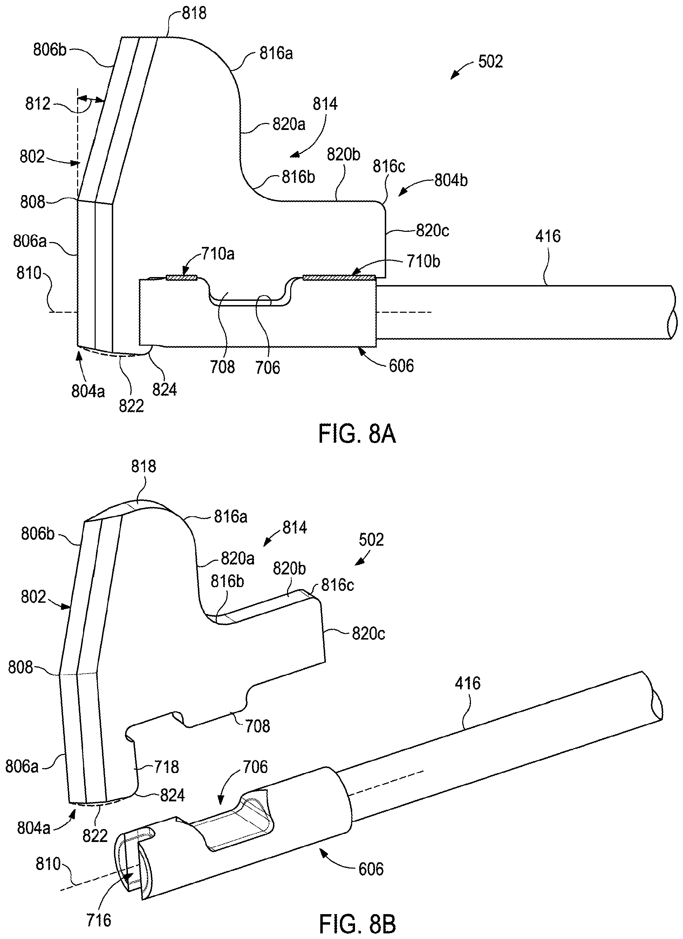

FIGS. 8A and 8B are side and exploded isometric views, respectively, of another example of the knife 502 as coupled to the distal end of the drive rod 416, according to one or more additional embodiments. The knife 502 shown in FIGS. 8A-8B may be similar in some respects to the knife 502 shown in FIGS. 7A-7B, and therefore may be best understood with reference thereto, where like numerals will correspond to like components not described again in detail.

Similar to the knife 502 of FIGS. 7A-7B, for example, the knife 502 of FIGS. 8A-8B includes the retention feature 608 secured to the distal end of the drive rod 416, and the central notch 706 is defined in the retention feature 608 and may extend partially into the underlying material of the drive rod 416. Moreover, the knife 502 provides the locking feature 708 sized to be received within the central notch 706, and the knife 502 can be secured to the outer surface of the retention feature 608 using one or both of the welds 710a,b (see FIG. 8A), as generally described above. Furthermore, the end tab 718 provided by the knife 502 may be received within the end notch 716 defined in the distal ends of the drive rod 416 and the retention feature 608.

Unlike the knife 502 of FIGS. 7A-7B, however, the knife 502 of FIGS. 8A-8B may include a dual-edge blade 802 provided at a distal or "leading" end 804a of the knife 502. The blade 802 is the sharp portion of the knife 502 opposite a proximal or "trailing" end 804b of the knife 502 and configured to sever or otherwise cut through tissue as the end effector 204 (FIGS. 2 and 4) fires and advances the knife 502 during operation. In the illustrated embodiment, the blade 802 may include a first or "leading" cutting edge 806a and a second or "angled" cutting edge 806b that extends from the leading cutting edge 806a at a transition point 808.

The leading cutting edge 806a may extend substantially orthogonal or perpendicular to a centerline 810 of the drive rod 416. Moreover, as explained in more detail below, the leading cutting edge 806b may also extend perpendicular to the sealing plane of the end effector 204 (FIGS. 2 and 4). As best seen in FIG. 8A, the angled cutting edge 806b extends from the leading cutting edge 806a at the transition point 808 and at an angle 812 offset from perpendicular to the centerline 810 (or the sealing plane of the end effector 204). The angle 812 may range between about 1° and about 60°, and any subset therebetween.

In some embodiments, the transition point 808 may comprise a sharp corner or transition between the leading and angled cutting edges 806a,b. In other embodiments, however, transition point may comprise an arcuate or curved transition between the leading an angled cutting edges 806a,b, without departing from the scope of the disclosure.

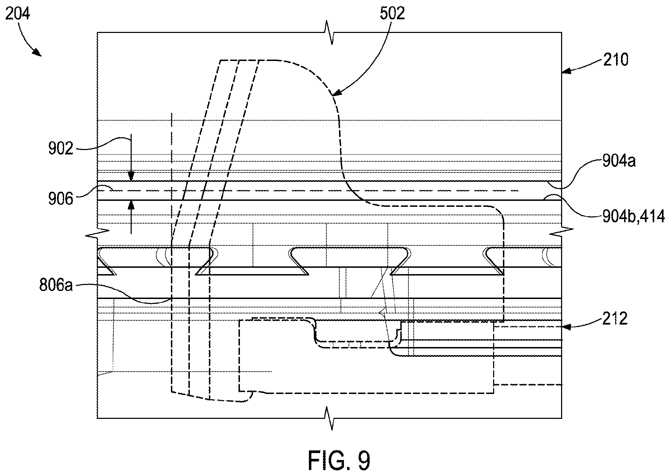

Referring briefly to FIG. 9, illustrated is an enlarged side view of the end effector 204 with the knife 502 during firing, according to one or more embodiments. More specifically, the end effector 204 is depicted in FIG. 9 in phantom, thus allowing a view of the knife 502 in the process of firing and otherwise being advanced distally (or proximally) within the knife slot 508 (FIG. 5).

As illustrated the end effector 204 includes the upper jaw 210 and the lower jaw 212, and a gap 902 is defined between the upper and lower jaws 210, 212 when the jaws 210, 212 are closed. The upper jaw 210 provides an upper surface 904a and the lower jaw 212 provides an opposing lower surface 904b. The lower surface 904b may be the same structure as the electrode 414 (FIGS. 4 and 5) attached to or forming part of the lower jaw 212. In some embodiments, the upper surface 904a may also comprise an electrode, but may otherwise comprise a planar sealing surface made of a nonconductive material (e.g., an insulator).

When the jaws 210, 212 are in the closed position, as shown in FIG. 9, the upper and lower surfaces 904a,b oppose each other and cooperatively form a sealing plane 906 that extends through the gap 902. The sealing plane 906 is alternately referred to as a "tissue capturing section" since the sealing plane 906 is the location where tissue can be grasped between the upper and lower jaws 210, 212 in preparation for cutting with the knife 502. As illustrated, as the knife 502 traverses the knife slot 508 (FIG. 5), the leading cutting edge 806a extends substantially perpendicular to the sealing plane 906.

In some embodiments, as illustrated, the entirety of the leading cutting edge 806a may be disposed below the sealing plane 906 and within the knife slot 508 (FIG. 5) defined in the lower jaw 212. In such embodiments, the transition point 808 may also be disposed below the sealing plane 906. In other embodiments, however, it is contemplated herein that the leading cutting edge 806a may extend to or above the sealing plane 906, thus also providing the transition point 808 at or above the sealing plane 906, without departing from the scope of the disclosure. In either scenario, the leading cutting edge 806a may be arranged and otherwise configured to cut tissue that may be located below the sealing plane 806, such as tissue that may migrate or "milk" into the knife slot 508 upon closing the jaws 210, 212. It is also contemplated herein to alter the arrangement of the leading cutting edge 806a and the angled cutting edge 806b such that the angled cutting edge 806b is positioned fully below the sealing plane 906, and only the leading cutting edge 806a is present in the sealing plane 906.

The leading cutting edge 806a being perpendicular to the sealing plane at 906 may also prove advantageous in providing a longer stroke length for the knife 502. More specifically, other knife designs, such as the knife 502 depicted in FIGS. 7A-7B, exhibit a fully angled cutting edge (extending top to bottom) that extends distally further than the distal location of the leading cutting edge 806a. As will be appreciated, this can limit how far the knife 502 can be advanced within the knife slot 508 (FIG. 5) since the angled bottom or "leading tip" of the fully angled cutting edge will engage the distal end of the knife slot 508. This can also result in breakage of the leading tip of the fully angled cutting edge by repeatedly engaging the distal end of the knife slot 508. Moreover, the leading tip can cause friction as the knife 502 traverses the curvature of the knife slot 508. In contrast, the leading cutting edge 806a extends perpendicular to the sealing plane 906 and thereby demonstrates a decrease in the active length of the knife 502, thus increasing potential overall cut length of the knife 502 along the knife slot 508.

Referring again to FIGS. 8A-8B, unlike the knife 502 of FIGS. 7A-7B, the trailing end 804b of the knife 502 of FIGS. 8A-8B may include or otherwise define a reduced trailing profile 814. The reduced trailing profile 814 may prove advantageous as the knife 502 traverses the knife slot 508 (FIG. 5). As shown in FIG. 5, the knife slot 508 is curved, and sometimes referred to as a Maryland-style knife track. Other knife designs, such as the knife 502 of FIGS. 7A-7B, provide or exhibit a full trailing profile that may catch or contact portions of the knife slot 508 provided in the upper jaw 210 (FIGS. 2 and 4) as the knife traverses the knife slot 508 when firing or retracting. Contacting the knife slot 508 during firing or retracting generates frictional loading and inefficiencies.

In contrast, the reduced trailing profile 814 of the knife 502 of FIGS. 8A-8B provides less surface area at the trailing end 804b and is therefore less prone to catch or contact portions of the knife slot 508 provided in the upper jaw 210 (FIGS. 2 and 4). More particularly, the reduced trailing profile 814 allows the knife 502 to navigate the curved knife slot 508 more easily and without significant frictional loading, including significant reduction in frictional drag when passing through tissue. Moreover, the reduced trailing profile 814 may lead to a significant reduction in sensitivity to cutting tissue in non-perpendicular orientations (e.g., cutting in the curvature of the knife slot 508, blade tilt, misalignment, etc.). The reduced trailing profile 814 also enables larger manufacturing tolerances due to less sensitivity. Lastly, the reduced trailing profile 814 may potentially reduce the amount of tissue particulate pulled back into the jaws 210, 212 (FIGS. 2 and 4), since there will be less tissue build-up.

In some embodiments, as illustrated, the reduced trailing profile 814 may include a first arcuate surface 816a extending from a top 818 of the knife 502, and the first arcuate surface 816a transitions to a second arcuate surface 816b, which then transitions to a third arcuate surface 816c. Accordingly, in at least one embodiment, the reduced trailing profile 814 may include three contiguous arcuate surfaces 816a-c. As illustrated, the first and third arcuate surfaces 816a,c may each be convex surfaces, and the second arcuate surface 816 be may be a concave surface.

In one or more embodiments, a first straight surface 820a may interpose the first and second arcuate surfaces 816a,b, and a second straight surface 820b may interpose the second and third arcuate surfaces 816b,c. In at least one embodiment, the first and second straight surfaces 820a,b may extend perpendicular to each other. Moreover, in at least one embodiment, a third straight surface 820c may extend from the third arcuate surface 816c, and the third straight surface 820c may extend generally parallel to the first straight surface 820a and perpendicular to the second straight surface 820b.

In some embodiments, the third straight surface 820c may extend substantially perpendicular to the centerline 810 of the drive rod 416. In other embodiments, however, the third straight surface 820c may extend at other angles relative to the centerline 180, without departing from the scope of the disclosure. In at least one embodiment the third straight surface 820c may be used to help positionally locate the knife 502 within the knife housing 506 (FIGS. 5 and 6A-6B) or to act as a hard stop or over-drive prevention feature. More specifically, as the knife 502 is retracted to the stowed position, as described above, the third straight surface 820c may be configured to engage a portion of the knife housing 506 within the cavity 602 (FIGS. 6A-6B). In other embodiments, however, as the knife 502 is retracted to the stowed position, the first straight surface 820a may alternatively be used to the knife housing for retraction, hard stops, over-drive prevention, and positional locating loads.

Still referring to FIGS. 8A-8B, in some embodiments, a bottom 822 (shown in dashed lines) of the knife 502 at the leading end 804a (e.g., on the end tab 718) may be curved and otherwise exhibit a radius. Having a curved bottom 822 may prove advantageous in helping to prevent the knife 502 from catching in the knife slot 508 (FIG. 5) when the knife 502 is moved in the distal direction (i.e., the "fire" direction). The curved bottom 822 may also result in less surface contact on the bottom of the knife 502 that might engage the bottom of the knife slot 508.

In some embodiments, the end tab 718 may define a curved proximal end 824, which may prove advantageous in helping to prevent the knife 502 from catching in the knife slot 508 (FIG. 5) when the knife 502 is moved in the distal direction (i.e., the "fire" direction). The curved proximal end 824 may also help reduce dragging of tissue and/or debris that may be present within the knife slot 508.

## Claims

1. An end effector (204) for a surgical tool (200), comprising:
opposing first and second jaws (210, 212), and a knife slot (508) defined in one or both of the first and second jaws;
a knife (502) extendable through the knife slot and operatively coupled to a drive rod (416) at a retention feature (608) secured to a distal end of the drive rod;
an end notch (716) defined in the distal end of the drive rod and extending through a distal end (704a) of the retention feature; and
an end tab (718) defined by the knife and sized to be received within the end notch to laterally stabilize the knife as mounted to the retention feature,
wherein a central notch (706) is defined in the retention feature between a proximal end (704b) of the retention feature and the distal end of the retention feature, wherein the central notch extends into the drive rod, and wherein the knife defines a locking feature (708) receivable within the central notch to axially constrain the knife to the retention feature and the drive rod.

2. The end effector of claim 1, wherein the retention feature is attached to the distal end of the drive rod via at least one of a crimped engagement, a welded interface, an adhesive attachment, an interference or shrink fit, an overmold, one or more mechanical fasteners, and any combination thereof.

3. The end effector of claim 1 or claim 2, wherein the locking feature is formed in the knife via at least one of, laser cutting, wire electrical discharge machining, milling, chemical etching, water jetting, and stamping.

4. The end effector of any preceding claim, wherein the locking feature extends into the central notch without contacting a bottom of the central notch such that a gap (712) is defined between the bottom of the central notch and the locking feature.

5. The end effector of any preceding claim, wherein the knife is welded to an outer surface of the retention feature.

6. The end effector of claim 5, wherein the knife is welded to the outer surface of the retention feature at a first weld (710a) located distal to the central notch, and a second weld (710b) located proximal to the central notch.

7. The end effector of claim 6, wherein a length of the distal weld is smaller than a length of the proximal weld.

8. The end effector of any preceding claim, wherein a length from a proximal end of the central notch to a proximal end of the retention feature is longer than a length from a distal end of the central notch to a distal end of the retention feature.

9. The end effector of any preceding claim, wherein the locking feature is received within the central notch such that a distal end (714b) of the locking feature contacts an opposing distal end (714a) of the central notch.

10. The end effector of any preceding claim, wherein a blade provided at a leading end of the knife includes:
a leading cutting edge (806a) extending perpendicular to a sealing plane (906) provided between the opposing first and second jaws when the opposing first and second jaws are closed; and
an angled cutting edge (806b) extending in a proximal direction from the leading cutting edge at a transition point (808), wherein the angled cutting edge extends from the leading cutting edge at an angle (812) offset from perpendicular to the sealing plane.

11. The end effector of claim 10, wherein the transition point is disposed below the sealing plane when the knife is advanced within the knife slot such that an entirety of the leading cutting edge is disposed in a portion of the knife slot defined in one of the first and second jaws.

12. The end effector of any preceding claim, wherein the knife provides a leading end (804a) and a trailing end (804b) opposite the leading end, and wherein a blade (802) is provided at the leading end and the trailing end defines a reduced trailing profile, the reduced trailing profile being defined by a first arcuate surface (816a) extending from a top (818) of the knife and transitioning to a second arcuate surface (816b) that transitions to a third arcuate surface (816c).

13. The end effector of claim 12, wherein the reduced trailing profile further includes:
a first straight surface (820a) interposing the first and second arcuate surfaces;
a second straight surface (820b) interposing the second and third arcuate surfaces; and
a third straight surface (820c) extending from the third arcuate surface.

14. The end effector of claim 13, wherein the third straight surface extends perpendicular to a centerline of the drive rod.

## Patentansprüche

1. Endeffektor (204) für ein chirurgisches Instrument (200), umfassend:
gegenüberliegende erste und zweite Backen (210, 212) und einen Messerschlitz (508), der in einer oder beiden der ersten und zweiten Backen definiert ist;
ein Messer (502), das durch den Messerschlitz ausfahrbar und an einem Haltemerkmal (608), die an einem distalen Ende der Antriebsstange befestigt ist, betriebsmäßig mit einer Antriebsstange (416) gekoppelt ist;
eine Endkerbe (716), die im distalen Ende der Antriebsstange definiert ist und sich durch ein distales Ende (704a) des Haltemerkmals erstreckt; und
eine Endlasche (718), die durch das Messer definiert ist und so bemessen ist, dass sie in die Endkerbe passt, um das Messer seitlich zu stabilisieren, wenn es am Haltemerkmal montiert ist,
wobei eine zentrale Kerbe (706) in dem Haltemerkmal zwischen einem proximalen Ende (704b) des Haltemerkmals und dem distalen Ende des Haltemerkmals definiert ist, wobei sich die zentrale Kerbe in die Antriebsstange erstreckt, und wobei das Messer eine Verriegelungsvorrichtung (708) definiert, die in der zentralen Kerbe aufgenommen werden kann, um das Messer axial an dem Haltemerkmal und der Antriebsstange zu befestigen.

2. Endeffektor nach Anspruch 1, wobei das Haltemerkmal am distalen Ende der Antriebsstange über mindestens eines der folgenden Mittel befestigt ist: eine Crimpverbindung, eine Schweißverbindung, eine Klebebefestigung, eine Press- oder Schrumpfpassung, eine Umspritzung, ein oder mehrere mechanische Befestigungselemente und eine beliebige Kombination davon.

3. Endeffektor nach Anspruch 1 oder 2, wobei die Verriegelungsfunktion im Messer durch mindestens eines der folgenden Verfahren gebildet wird: Laserschneiden, Drahterodierbearbeitung, Fräsen, chemisches Ätzen, Wasserstrahlen und Stanzen.

4. Endeffektor nach einem der vorstehenden Ansprüche, wobei sich das Verriegelungsmerkmal in die zentrale Kerbe erstreckt, ohne einen Boden der zentralen Kerbe zu berühren, so dass zwischen dem Boden der zentralen Kerbe und dem Verriegelungsmerkmal ein Spalt (712) definiert ist.

5. Endeffektor nach einem der vorstehenden Ansprüche, wobei das Messer an eine Außenfläche des Haltemerkmals geschweißt ist.

6. Endeffektor nach Anspruch 5, wobei das Messer an einer ersten Schweißnaht (710a), die distal zur zentralen Kerbe liegt, und einer zweiten Schweißnaht (710b), die proximal zur zentralen Kerbe liegt, an die Außenfläche des Haltemerkmals geschweißt ist.

7. Endeffektor nach Anspruch 6, wobei eine Länge der distalen Schweißnaht kleiner ist als eine Länge der proximalen Schweißnaht.

8. Endeffektor nach einem der vorstehenden Ansprüche, wobei eine Länge von einem proximalen Ende der zentralen Kerbe zu einem proximalen Ende des Haltemerkmals länger ist als eine Länge von einem distalen Ende der zentralen Kerbe zu einem distalen Ende des Haltemerkmals.

9. Endeffektor nach einem der vorstehenden Ansprüche, wobei das Verriegelungsmerkmal so in der zentralen Kerbe aufgenommen ist, dass ein distales Ende (714b) des Verriegelungsmerkmals ein gegenüberliegendes distales Ende (714a) der zentralen Kerbe berührt.

10. Endeffektor nach einem der vorstehenden Ansprüche, wobei eine an einem vorderen Ende des Messers vorgesehene Klinge Folgendes einschließt:
eine vordere Schneidkante (806a), die sich senkrecht zu einer Versiegelungsebene (906) erstreckt, die zwischen den gegenüberliegenden ersten und zweiten Backen vorgesehen ist, wenn die gegenüberliegenden ersten und zweiten Backen geschlossen sind; und
eine abgewinkelte Schneidkante (806b), die sich an einem Übergangspunkt (808) von der vorderen Schneidkante in proximaler Richtung erstreckt, wobei sich die abgewinkelte Schneidkante von der vorderen Schneidkante in einem Winkel (812) erstreckt, der von der Senkrechten zur Versiegelungsebene versetzt ist.

11. Endeffektor nach Anspruch 10, wobei der Übergangspunkt unterhalb der Versiegelungsebene angeordnet ist, wenn das Messer innerhalb des Messerschlitzes vorgeschoben wird, so dass die gesamte vordere Schneidkante in einem Abschnitt des Messerschlitzes angeordnet ist, der in einer der ersten und zweiten Backen definiert ist.

12. Endeffektor nach einem der vorstehenden Ansprüche, wobei das Messer ein vorderes Ende (804a) und ein dem vorderen Ende gegenüberliegendes hinteres Ende (804b) aufweist, und wobei am vorderen Ende eine Klinge (802) bereitgestellt ist und das hintere Ende ein reduziertes hinteres Profil definiert, wobei das reduzierte hintere Profil durch eine erste bogenförmige Oberfläche (816a) definiert ist, die sich von einer Oberseite (818) des Messers erstreckt und in eine zweite bogenförmige Oberfläche (816b) übergeht, die in eine dritte bogenförmige Oberfläche (816c) übergeht.

13. Endeffektor nach Anspruch 12, wobei das reduzierte hintere Profil ferner einschließt:
eine erste gerade Oberfläche (820a), die zwischen der ersten und der zweiten bogenförmigen Oberfläche liegt;
eine zweite gerade Oberfläche (820b), die zwischen der zweiten und dritten bogenförmigen Oberfläche liegt; und
eine dritte gerade Oberfläche (820c), die sich von der dritten bogenförmigen Oberfläche erstreckt.

14. Endeffektor nach Anspruch 13, wobei die dritte gerade Oberfläche senkrecht zu einer Mittellinie der Antriebsstange verläuft.

## Revendications

1. Effecteur terminal (204) destiné à un outil chirurgical (200), comprenant :
des première et seconde mâchoires (210, 212) opposées, et une fente de couteau (508) définie dans l'une ou les deux parmi les première et seconde mâchoires ;
un couteau (502) extensible à travers la fente de couteau et accouplé de manière fonctionnelle à une tige d'entraînement (416) au niveau d'un élément de rétention (608) fixé à une extrémité distale de la tige d'entraînement ;
une encoche d'extrémité (716) définie dans l'extrémité distale de la tige d'entraînement et s'étendant à travers une extrémité distale (704a) de l'élément de rétention ; et
une languette d'extrémité (718) définie par le couteau et dimensionnée afin d'être reçue au sein de l'encoche d'extrémité afin de stabiliser latéralement le couteau lorsqu'il est monté sur l'élément de rétention,
dans lequel une encoche centrale (706) est définie dans l'élément de rétention entre une extrémité proximale (704b) de l'élément de rétention et l'extrémité distale de l'élément de rétention, dans lequel l'encoche centrale s'étend dans la tige d'entraînement, et dans lequel le couteau définit un élément de verrouillage (708) pouvant être reçu au sein de l'encoche centrale afin de contraindre axialement le couteau à l'élément de rétention et à la tige d'entraînement.

2. Effecteur terminal selon la revendication 1, dans lequel l'élément de rétention est fixé à l'extrémité distale de la tige d'entraînement par le biais d'au moins l'un parmi une mise en prise sertie, une interface soudée, une fixation adhésive, un ajustement serré ou fretté, un surmoulage, une ou plusieurs attaches mécaniques, et une quelconque combinaison de ceux-ci.

3. Effecteur terminal selon la revendication 1 ou la revendication 2, dans lequel l'élément de verrouillage est formé dans le couteau par le biais d'au moins l'un parmi une découpe laser, un usinage par décharge électrique de fil, un fraisage, une gravure chimique, un jet d'eau, et un estampage.

4. Effecteur terminal selon l'une quelconque revendication précédente, dans lequel l'élément de verrouillage s'étend dans l'encoche centrale sans entrer en contact avec un fond de l'encoche centrale de telle sorte qu'un trou (712) est défini entre le fond de l'encoche centrale et l'élément de verrouillage.

5. Effecteur terminal selon l'une quelconque revendication précédente, dans lequel le couteau est soudé à une surface externe de l'élément de rétention.

6. Effecteur terminal selon la revendication 5, dans lequel le couteau est soudé à la surface externe de l'élément de rétention au niveau d'une première soudure (710a) située à distance de l'encoche centrale, et d'une seconde soudure (710b) située à proximité de l'encoche centrale.

7. Effecteur terminal selon la revendication 6, dans lequel une longueur de la soudure distale est inférieure à une longueur de la soudure proximale.

8. Effecteur terminal selon l'une quelconque revendication précédente, dans lequel une longueur à partir d'une extrémité proximale de l'encoche centrale jusqu'à une extrémité proximale de l'élément de rétention est plus longue qu'une longueur à partir de l'extrémité distale de l'encoche centrale jusqu'à l'extrémité distale de l'élément de rétention.

9. Effecteur terminal selon l'une quelconque revendication précédente, dans lequel l'élément de verrouillage est reçu au sein de l'encoche centrale de telle sorte qu'une extrémité distale (714b) de l'élément de verrouillage entre en contact avec une extrémité distale (714a) opposée de l'encoche centrale.

10. Effecteur terminal selon l'une quelconque revendication précédente, dans lequel une lame fournie au niveau d'une extrémité avant du couteau comporte :
un bord de coupe avant (806a) s'étendant perpendiculairement à un plan d'étanchéification (906) fourni entre les première et seconde mâchoires opposées lorsque les première et seconde mâchoires opposées sont fermées ; et
une arête de coupe angulaire (806b) s'étendant dans une direction proximale à partir de l'arête de coupe avant au niveau d'un point de transition (808), dans lequel l'arête de coupe angulaire s'étend à partir de l'arête de coupe avant selon un angle (812) décalé perpendiculairement au plan d'étanchéification.

11. Effecteur terminal selon la revendication 10, dans lequel le point de transition est disposé sous le plan d'étanchéification lorsque le couteau est avancé au sein de la fente de couteau de sorte qu'une totalité du bord de coupe avant est disposée dans une partie de la fente de couteau définie dans l'une parmi les première et seconde mâchoires.

12. Effecteur terminal selon l'une quelconque revendication précédente, dans lequel le couteau fournit une extrémité avant (804a) et une extrémité arrière (804b) opposée à l'extrémité avant, et dans lequel une lame (802) est fournie au niveau de l'extrémité avant et l'extrémité arrière définit un profil arrière réduit, le profil arrière réduit étant défini par une première surface arquée (816a) s'étendant à partir d'un sommet (818) du couteau et transitionnant vers une deuxième surface arquée (816b) qui transitionne vers une troisième surface arquée (816c).

13. Effecteur terminal selon la revendication 12, dans lequel le profil arrière réduit comporte en outre :
une première surface droite (820a) interposant les première et deuxième surfaces arquées ;
une deuxième surface droite (820b) interposant les deuxième et troisième surfaces arquées ; et
une troisième surface droite (820c) s'étendant à partir de la troisième surface arquée.

14. Effecteur terminal selon la revendication 13, dans lequel la troisième surface droite s'étend perpendiculairement à une ligne médiane de la tige d'entraînement.
